# EUROPEAN PATENT APPLICATION

(11) **EP 4 563 172 A1**
(43) Date of publication of application: **04.06.2025**
(21) Application number: 23860622.2
(22) Date of filing: 26.06.2023
(51) Int. Cl.: A61M 5/145, A61M 5/172, A61M 5/142, G16H 20/17, G16H 50/20, G16H 40/63

(54) **DRUG INJECTION DEVICE AND METHOD**

(30) Priority: 29.08.2022 KR 20220108095; 07.06.2023 KR 20230072555
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR); SHIN, Yeong Jong, Gimpo-si Gyeonggi-do 10126 (KR)
(74) Representative: Manitz Finsterwald Patent- und Rechtsanwaltspartnerschaft mbB
(86) International application number: PCT/KR2023/008821
(87) International publication number: WO 2024/048938

(57) **Abstract**

A drug injection device includes a drug injector configured to suck a drug from storage by operating an electroosmotic pump and discharge it to an injection target; and a controller configured to output to the injector a control signal for a speed-based injection sequence, causing the electroosmotic pump to operate in basal infusion mode. The speed-based injection sequence includes at least one pulse block defining a voltage or current pulse applied to the pump and at least one pause block maintaining 0 volt or 0 ampere for a predetermined time after the pulse block. The pulse block defines a pair of signals, a forward and reverse pulse, applied to the electroosmotic pump to alternately generate negative and positive pressure, enabling drug suction and discharge.

## Description

### BACKGROUND

The present invention relates to a drug injection device and method.

Drugs may be injected into the body in various ways, such as oral, subcutaneous, and intravenous administration, depending on the type, purpose of treatment, and method. A drug injection device using a drug pump may automatically inject drugs into the body at a desired speed and dose at a required time. Therefore, a drug injection device using a drug pump may be used not only in hospitals or in patients' daily living environments, but also in various forms.

An insulin pump, which is commonly called an insulin injector, is a medical device that supplies insulin into the body from the outside at a set time to accurately control blood sugar levels, similar to a pancreas, for diabetic patients who do not secrete insulin or secrete only a small amount of insulin.

Such an insulin pump is used by insulin-dependent diabetic patients, and may continuously inject drugs for 24 hours in a state of being attached to the patient. In this way, an insulin injector has to periodically inject drugs for a long period of time for diabetic patients, and accordingly, active technology development is being conducted to miniaturize and automate insulin injectors for the convenience of users.

However, as insulin injectors are miniaturized and automated, there are cases where it is difficult to actually secure the stability of the operation of the insulin injector, and this is mostly due to the back pressure caused by various reactions between the drug and the biological fluid at the tip of the cannula or needle, which is the main cause of the blockage phenomenon. In particular, when the amount of injected drug is small or is injected slowly, the blockage phenomenon becomes more serious.

Therefore, a method of continuously inject drugs into patients, stably inject an accurate amount of drugs, and control the injection of drugs such that a path is not blocked during the drug injection is required.

The present invention provides a drug injection method for basal injection by a drug injection device based on an electroosmotic pump.

However, technical issues to be solved by the present embodiments are not limited to the technical issues described above, and other technical issues may be derived therefrom.

### SUMMARY

A drug injection device according to an embodiment of the present invention includes a drug injector configured to suck a drug from a drug storage by electrochemically operating an electroosmotic pump and discharge an sucked drug to an injection target; and a controller configured to output, to the drug injector, a control signal corresponding to a speed-based injection sequence for causing the electroosmotic pump to operate in a basal infusion mode, wherein the speed-based injection sequence includes at least one pulse block defining one of a voltage pulse and a current pulse applied to the electroosmotic pump and at least one pause block in which one of a voltage of 0 volt and a current of 0 ampere is maintained for a predetermined period of time after the pulse block is applied, the at least one pulse block defines a pair of pulse signals including a forward pulse and a reverse pulse that are applied to the electroosmotic pump to alternately generate negative pressure and positive pressure, and the electroosmotic pump alternately generates negative pressure and positive pressure for each pulse block to suck and discharge the drug.

A drug injection method according to an embodiment of the present invention includes setting a speed-based injection sequence for causing the drug injection device to operate in a basal infusion mode; applying a control signal corresponding to the speed-based injection sequence to the electroosmotic pump; and causing the electroosmotic pump to alternately generate negative pressure and positive pressure for each pulse block in response to the control signal such that the drug is sucked and discharged, wherein the speed-based injection sequence includes at least one pulse block defining one of a voltage pulse and a current pulse applied to the electroosmotic pump and at least one pause block in which one of a voltage of 0 volt and a current of 0 ampere is maintained for a predetermined period of time after the pulse block is applied, and the at least one pulse block defines a pair of pulse signals including a forward pulse and a reverse pulse that are applied to the electroosmotic pump to alternately generate the negative pressure and the positive pressure.

According to the present invention described above, a drug injector using an electroosmotic pump may inject a fixed amount of drug by setting a speed-based injection sequence according to drug injection conditions.

### BRIEF DESCRIPTION OF THE DRAWINGS

FIG. 1 is a block diagram schematically illustrating a drug injection device according to an embodiment of the present invention.
FIG. 2 is a block diagram schematically illustrating a configuration of a drug injector illustrated in FIG. 1.
FIG. 3 is a conceptual diagram schematically illustrating a speed-based injection sequence according to an embodiment of the present invention.
FIG. 4 is a table showing an example of multiple speed-based injection sequences according to an embodiment of the present invention.
FIG. 5 is a table showing an example of multiple pulse blocks according to an embodiment of the present invention.
FIG. 6 is a table showing an example of a drug injection period according to an embodiment of the present invention.
FIG. 7 is an example diagram illustrating a signal structure for a speed-based injection sequence according to an embodiment of the present invention.
FIG. 8 is a block diagram schematically illustrating a configuration of an electroosmotic pump illustrated in FIG. 2.
FIG. 9 is a block diagram schematically illustrating a configuration of a driver illustrated in FIG. 8.
FIG. 10 is an example diagram schematically illustrating a configuration of a driver illustrated in FIG. 9.
FIG. 11 is an application example diagram of a drug injection device according to an embodiment of the present invention.
FIG. 12 is a block diagram schematically illustrating a configuration of the drug injection device illustrated in FIG. 11.
FIG. 13 is a block diagram schematically illustrating a configuration of the drug injection device illustrated in FIG. 11.
FIG. 14 is a flowchart illustrating a drug injection method according to an embodiment of the present invention.

### DETAILED DESCRIPTION OF THE EMBODIMENTS

Hereafter, the present disclosure is described in detail with reference to the accompanying drawings. However, the present disclosure may be implemented in many different forms and is not limited to the embodiments described herein. In addition, the accompanying drawings are only for easy understanding of the embodiments disclosed in the present specification, and the technical ideas disclosed in the present specification are not limited by the accompanying drawings. In order to clearly describe the present disclosure in the drawings, parts irrelevant to the descriptions are omitted, and a size, a shape, and a form of each component illustrated in the drawings may be variously modified. The same or similar reference numerals are assigned to the same or similar portions throughout the specification.

Suffixes "module" and "unit" for the components used in the following description are given or used interchangeably in consideration of ease of writing the specification, and do not have meanings or roles that are distinguished from each other by themselves. In addition, in describing the embodiments disclosed in the present specification, when it is determined that a detailed descriptions of related known technologies may obscure the gist of the embodiments disclosed in the present specification, the detailed descriptions are omitted.

Throughout the specification, when a portion is said to be "connected (coupled, in contact with, or combined)" with another portion, this includes not only a case where it is "directly connected (coupled, in contact with, or combined)" ", but also a case where there is another member therebetween. In addition, when a portion "includes (comprises or provides)" a certain component, this does not exclude other components, and means to "include (comprise or provide)" other components unless otherwise described.

Terms indicating ordinal numbers, such as first and second, used in the present specification are used only for the purpose of distinguishing one component from another component and do not limit the order or relationship of the components. For example, the first component of the present disclosure may be referred to as the second component, and similarly, the second component may also be referred to as the first component.

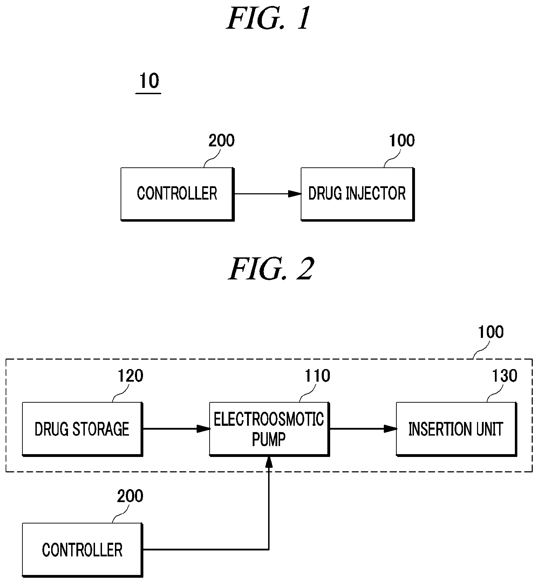

FIG. 1 is a block diagram schematically illustrating a configuration of a drug injection device according to an embodiment of the present invention, and FIG. 2 is a block diagram schematically illustrating a configuration of the drug injection device illustrated in FIG. 1.

Referring to FIG. 1 and FIG. 2, a drug injection device 10 according to an embodiment of the present invention includes a drug injector 100 and a controller 200.

The drug injector 100 sucks a drug from a drug storage by electrochemically operating an electroosmotic pump 110 in a basal injection mode and discharges the sucked drug to an injection target. In addition, the controller 200 outputs a control signal corresponding to a speed-based injection sequence for causing the drug injector 100 to operate the electroosmotic pump 110 in the basal injection mode. Here, the basal injection mode is a drug injection mode in which a drug is injected into a user for a certain period of time to maintain a constant blood sugar level of the user.

The controller 200 may mean a data processing device which is embedded in hardware and includes a physically structured circuit to perform a function expressed as a code or command included in a program. The data processing device embedded in hardware may include, for example, a microprocessor, a central processing unit (CPU), a processor core, a multiprocessor, an application-specific integrated circuit (ASIC), a field programmable gate array (FPGA), a micro control unit (MCU), an embedded processor, or so on, but the scope of the present invention is not limited thereto.

Meanwhile, the controller 200 may be embedded solely in the drug injection device 10, or may control the basal injection mode of the drug injection device 10 by being connected to a user terminal 40 through a communication module and being integrated into the user terminal 40.

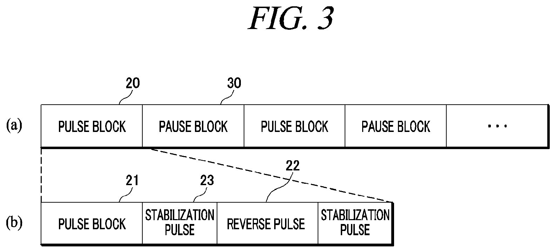

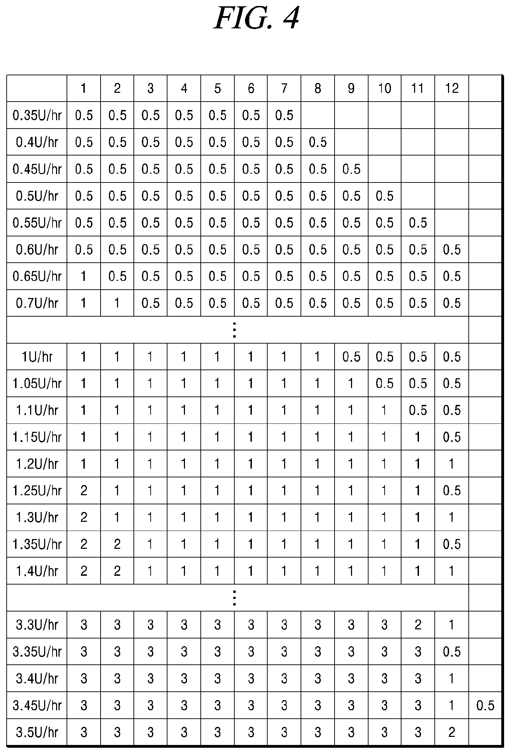

FIG. 3 is a diagram illustrating a concept of a speed-based injection sequence according to an embodiment of the present invention, and FIG. 4 to FIG. 7 are example diagrams illustrating a speed-based injection sequence according to an embodiment of the present invention. Hereinafter, the speed-based injection sequence is specifically described with reference to FIG. 3 to FIG. 7.

The speed-based injection sequence includes multiple pulse blocks 20 that satisfy a drug injection speed according to a drug injection condition including a drug injection period and a drug injection speed. In this case, the speed-based injection sequence is configured by arranging the multiple pulse blocks that satisfy the drug injection speed for a unit time (for example, 1 hour or 30 minutes) and repeating the multiple pulse blocks during the drug injection period.

Referring to FIG. 3 (a), the speed-based injection sequence includes the multiple pulse blocks 20 and multiple pause block 30 and starts with the pulse block 20, and the pause block 30 is arranged after the pulse block 20. The pulse block 20 defines a voltage pulse or a current pulse to be applied to the electroosmotic pump 110, and the amount of injected drugs changes depending on a duration. The pause block 30 maintains a voltage of 0 volt or a current of 0 ampere for a predetermined time after the pulse block 20 is applied.

In order to satisfy the drug injection condition including the drug injection period and drug injection speed, the speed-based injection sequence is set as follows. First, in order to satisfy the drug injection speed, a pulse block satisfying the drug injection amount per unit time has to be arranged. In the present invention, various pulse blocks may be set by the electroosmotic pump 110. However, during the drug injection period, multiple or preset pulse blocks may be arranged to reduce a pause period of the pause block arranged after the respective pulse blocks, and accordingly, drug may not be dried out or may not be blocked. In order to satisfy the condition, the speed-based injection sequence may be set as follows.

In the speed-based injection sequence, M pulse blocks 20 (M is a natural number less than or equal to N that is a natural number) selected from among N pulse blocks 20 that supply different amounts of drugs are arranged according to the drug injection speed for a unit time. The multiple pulse blocks 20 and the multiple pause block 30 that constitute the speed-based injection sequence are configured as a combination that satisfies the drug injection speed while the duration of each of the multiple pause blocks 30 is less than a recommended pause period. Here, the number of pulse blocks 20 included in the speed-based injection sequence may be a preset number according to the unit time or the maximum number that may represent the drug injection speed.

In addition, the sum of durations of the multiple pulse blocks 20 included in the speed-based injection sequence and durations of the multiple pause blocks 30 arranged after the pulse blocks 20 may be set to correspond to the unit time, and in arranging the multiple pulse blocks 20, the pulse block 20 supplying the largest amount of drugs is arranged to be output first, and subsequent arrangement of the pulse blocks 20 may be changed depending on options.

In addition, the multiple pulse blocks 20 included in the speed-based injection sequence may be set such that the sum of the number of uses for each selected pulse block 20 is a preset number or a maximum number while satisfying the drug injection speed of the drug injection condition, and durations of the pause blocks 30 arranged after each pulse block 20 may also be set to be less than the recommended pause period.

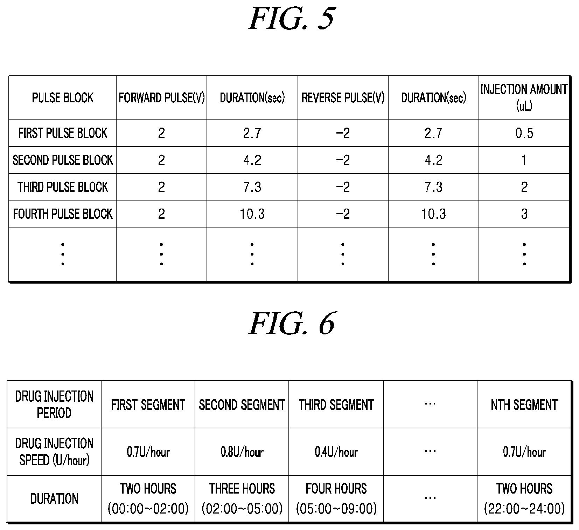

FIG. 4 is a table showing an example of multiple speed-based injection sequences, and FIG. 5 is a table showing an example of N pulse blocks 20 for injecting different amounts of drugs. Multiple speed-based injection sequences are described with reference to FIG. 4 and FIG. 5 by using examples.

The multiple speed-based injection sequences illustrated in FIG. 4 are set to different drug injection speeds. The speed-based injection sequence is composed of a combination of multiple pulse blocks 20 that may be set such that durations of the multiple pause blocks 30 are less than the recommended pause period while satisfying the drug injection speed among the multiple pulse blocks 20 that supply different amounts of drugs illustrated in FIG. 5. In addition, each speed-based injection sequence is arranged such that the pulse block 20 which supplies the largest amount of drugs among the multiple pulse blocks 20 that constitute each speed-based injection sequence is output first.

Here, the speed-based injection sequence is set such that 12 pulse blocks 20 may be arranged and is composed of a combination of pulse blocks 20 that may satisfy the drug injection speed while 12 pulse blocks 20 are all arranged. For example, a speed-based injection sequence with a drug injection speed of 0.7 U/hr is composed of a combination of two pulse blocks 20 that respectively supply 0.5 µL and 1 µL, and the pulse block 20 that supplies 1 µL is arranged first. In addition, the sum of the number of uses of each of the multiple pulse blocks 20 that supply 0.5 µL and 1 µL is a preset number of 12. Here, in FIG. 4, the drug injection amount of the speed-based injection sequence is represented in U units, and in FIG. 5, the drug injection amount of the pulse block 20 is represented in µ units, and 1 U is equal to 10 u. Therefore, 0.35 U is equal to 3.5 µ.

In addition, although not illustrated in the table, pause blocks 30 are all arranged after the pulse blocks 20, and durations of the pause blocks 30 are set to be less than the recommended duration to prevent blocking. The duration of the pause block 30 is set differently according to the duration of the pulse block 20 arranged immediately before the pause block 30.

For example, in a process of setting the duration of the idle block 30 in FIG. 4, each speed-based injection sequence has a structure in which 12 pulse blocks 20 are arranged, and the duration of one pulse block 20 and one pause block 30 is set to 300 seconds (3600 seconds/12). For example, the duration of the pause block 30 arranged after the pulse block 20 that injects 0.5 µL is the time obtained by subtracting the duration of the pulse block 20 that injects 0.5 µL from 300 seconds, and referring to FIG. 5, the duration of the pulse block 20 that injects 0.5 µL is 5.4 seconds, which is the sum of a duration of a forward pulse and a duration of the reverse pulse, and accordingly, the duration of the pause block 30 is set to 294.6 seconds, which is obtained by subtracting 5.4 seconds from 300 seconds.

Additionally, the speed-based injection sequence may not have a combination of pulse blocks 20 that satisfy the preset number. The number may be less than or more than the preset number. In FIG. 4, the speed-based injection sequence having a drug injection speed of 0.35 U/hr to 0.55 U/hr is configured with less than 12 pulse blocks 20. In this case, the pulse blocks 20 are configured with the maximum number that may be arranged among the number less than the preset number to satisfy the drug injection speed. In addition, when the speed-based injection sequence has a speed of 3.45 U/hr, the number of pulse blocks 20 is 13 which exceeds 12, and in this case, the structure of the speed-based injection sequence may be set again to a structure in which 13 pulse blocks 20 are arranged per hour, and accordingly, the pulse blocks 20 and pause blocks 30 may be arranged.

In addition, although the structure of the speed-based injection sequence having the unit time set to 1 hour is illustrated as an example in FIG. 4, the present invention is not limited thereto, and the number and structure of the pulse blocks 20 constituting the speed-based injection sequence may change depending on the set unit times.

The speed-based injection sequences are sequentially arranged during a drug injection period. Specifically, the drug injection period may be divided into at least one segment, and each segment includes information on the duration and drug injection speed in the duration. Each segment may be configured by repeatedly arranging speed-based injection sequences set for a unit time to satisfy a drug injection speed during a duration. Here, the duration and drug injection speed of each segment may be set by a user.

Referring to FIG. 6, an example of a drug injection period configured with a speed-based injection sequence is described. FIG. 6 illustrates a state in which the drug injection period is set to 24 hours, and 24 hours are divided into multiple segments to which a predetermined time zone is assigned. In a structure of the first segment, the first segment is a section in which a drug is injected at a rate of 0.7 U/hour for 2 hours, and two speed-based injection sequences, each having a drug injection speed of 0.7 U/hour illustrated in FIG. 4, are sequentially arranged. Here, each segment is set to a 1-hour unit, but the present invention is not limited thereto, and each segment may be distinguished according to a preset unit time.

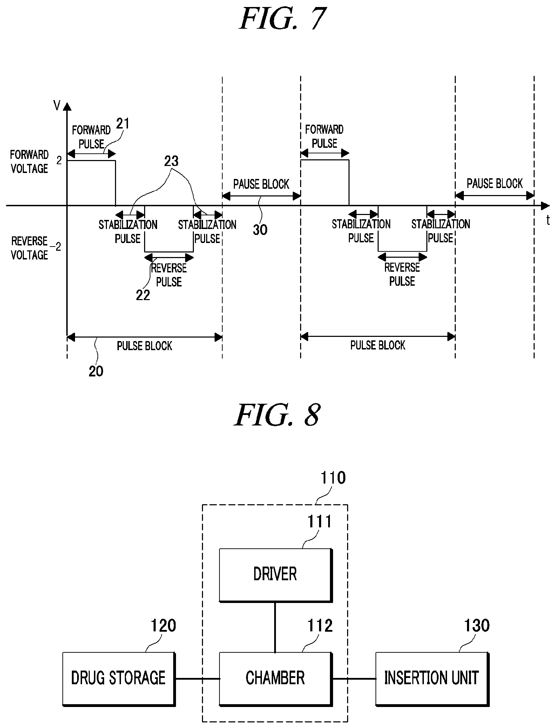

Next, referring to FIG. 3 (b) and FIG. 7, each of the pulse blocks 20 includes information on a pair of pulse signals including a forward pulse and a reverse pulse, and includes information on an amplitude of each pulse and a duration for which a pulse is maintained. The pair of pulse signals including the forward pulse and the reverse pulse are applied to the electroosmotic pump 110 to alternately generate negative pressure and positive pressure, and thereby, the negative pressure and positive pressure are alternately generated in each of the pulse blocks 20 to suck and discharge a drug.

A pair of pulse signals 21 and 22 included in the pulse block 20 is a voltage pulse signal or a current pulse signal. A pair of voltage pulse signals may include a forward voltage pulse and a reverse voltage pulse, and a pair of current pulse signals may include a forward current pulse and a reverse current pulse. Here, the pair of voltage pulse signals may include information on amplitudes and durations of the respective voltage pulses, and the pair of current pulse signals may include information on amplitudes and durations of the respective current pulses. For example, in the speed-based injection sequence illustrated in FIG. 7, the amplitude of a pulse signal may be 2 volts, and the duration thereof may be 10 seconds.

Pulse blocks 20 included in the speed-based injection sequence may each include voltage pulses having different voltage levels or current pulses having different current levels. Alternatively, the pulse blocks 20 may each include pulses having the same voltage level or current pulses having the same current level, but respective durations may be set differently.

In addition, the forward pulse 21 and the reverse pulse 22 included in the pulse block 20 may be set to have the same amplitude and duration, and accordingly, amounts of drugs sucked and discharged by the electroosmotic pump 110 may be maintained to be the same.

In addition, the pair of pulse signals 21 and 22 included in the pulse block 20 may be provided as a constant voltage or a constant current, and the amount of drugs sucked and discharged by each pulse block may be adjusted by controlling the duration of a pulse signal provided by the constant voltage or the constant current. For example, the pulse blocks 20 of FIG. 4 are provided by a constant voltage of 2 volts.

Additionally, the pair of pulse signals 21 and 22 may be controlled in both amplitudes and durations, and accordingly, the amounts of drugs sucked and discharged may be adjusted to be the same. For example, an amplitude of the forward voltage pulse is 2 volts, the duration is 10 seconds, and the amplitude of a subsequent reverse voltage pulse is set to 1 volt, and the duration thereof is 20 seconds, and accordingly, an area of the forward pulse and an area of the reverse pulse are set to equal to each other to maintain the amount of sucked drug and the amount of discharged drug to be equal to each other.

In addition, the pulse block 20 may further include a stabilization pulse 23, and the stabilization pulse 23 is a pulse that maintains a voltage of 0 volt for a predetermined time after the forward voltage pulse 21 and the reverse voltage pulse 22 are applied. An operation of the electroosmotic pump 110 may be stabilized by the stabilization pulse 23. Here, when the pulse block 20 of the speed-based injection sequence includes a pair of current pulses, the pulse block 20 may further include the stabilization pulse 23 that maintains a current of 0 ampere for a predetermined time after the forward current pulse and the reverse current pulse are applied.

Subsequently, the pause block 30 is arranged after the pulse block 20 is applied, and is a signal that a voltage of 0 volt or a current of 0 ampere continues for a predetermined time. The drug injection device 10 of the present invention injects a drug to a user by a certain amount of drugs for a predetermined time. Therefore, the time at which a target drug injection amount is injected may be controlled by the pause block 30 included in the speed-based injection sequence, and by reducing a time period during which a drug is not injected, it is possible to prevent a flow path of the drug injector 100 from being blocked during a time period during which a drug is not injected.

The controller 200 generates a control signal including a voltage pulse or a current pulse corresponding to the speed-based injection sequence based on information of the pulse block 20 included in the speed-based injection sequence and applies the control signal to the drug injector 100.

When a control signal corresponding to the speed-based injection sequence is applied to the electroosmotic pump 110, the electroosmotic pump 110 alternately generates negative pressure and positive pressure for each pulse block to suck and discharge drugs. Here, the control signal may be a signal including a voltage pulse pair including a forward voltage pulse and a reverse voltage pulse in units of the pulse block 20 or a current pulse pair including a forward current pulse and a reverse current pulse in units of pulse blocks.

FIG. 8 is a block diagram schematically illustrating a configuration of the electroosmotic pump 110 illustrated in FIG. 2.

Referring to FIG. 8, the electroosmotic pump 110 includes a driver 111 and a chamber 112. The driver 111 is electrochemically driven by a control signal to generate positive pressure and negative pressure and discharges a drug according to the positive pressure and negative pressure, and the chamber 112 sucks a drug from a drug storage 120 according to the pressure of the driver 111 and then discharges the drug to an insertion unit 130. Here, the drug is a drug that has to be injected into a certain patient and may be, for example, insulin that is injected into a diabetic patient.

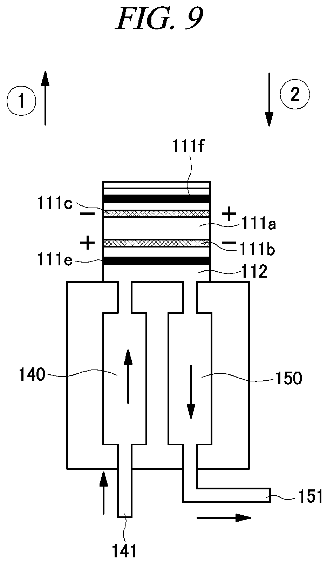

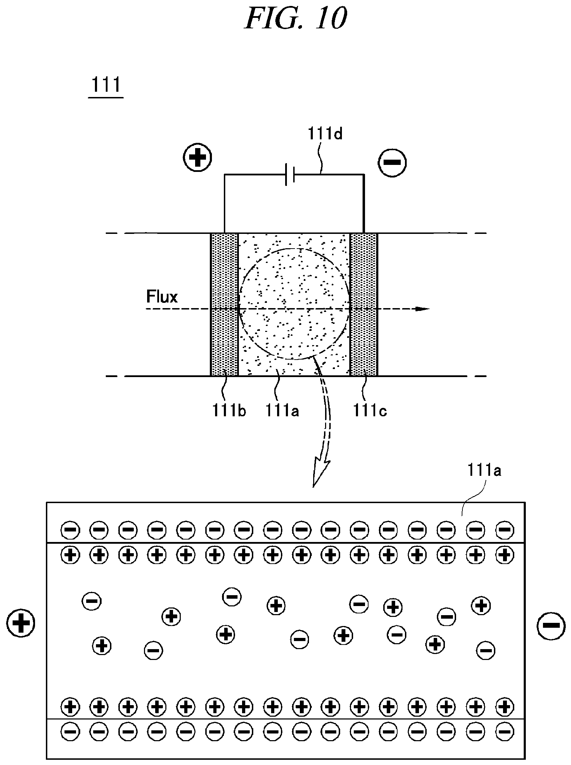

FIG. 9 is a block diagram schematically illustrating a configuration of the driver 111 illustrated in FIG. 8, and FIG. 10 is an example diagram schematically illustrating a configuration of the driver 111 illustrated in FIG. 9.

Referring to FIG. 9 and FIG. 10, the driver 111 is a pump that uses the movement of fluid according to an electroosmotic phenomenon that occurs when a voltage or current is applied by using electrodes at both ends of a porous film (membrane) 111a. The driver 111 may include the membrane 111a, a power source 111d that applies a voltage or current to a first electrode 111b and a second electrode 111c arranged on both sides of the membrane 111a, and a flow path through which a fluid moves.

In addition, the driver 111 may include a first diaphragm 111e arranged adjacent to the first electrode 111b and a second diaphragm 111f arranged adjacent to the second electrode 111c. The first and second diaphragms 111e and 111f are provided respectively one side and the other side of the membrane 111a, and shapes of the first and second diaphragms 111e and 111f are deformed by the movement of a pumping solution as positive pressure and negative pressure are alternately generated. For example, the first diaphragm 111e and the second diaphragm 111f transfer the negative pressure and positive pressure generated by an operation of the membrane 111a to a transfer target fluid. More specifically, when negative pressure is generated, at least a part of the first diaphragm 111e and the second diaphragm 111f moves backward (moving in a direction ①) such that the transfer target fluid is sucked to the chamber 112, and in contrast to this, when positive pressure is generated, at least a part of the first diaphragm 111e and the second diaphragm 111f moves forward (moving in a direction ②) such that the transfer target fluid is discharged from the chamber 112.

Silica, glass, and so on are generally used as a material of the porous film 111a, and when the silica and glass are immersed in an aqueous solution, surfaces thereof are negatively charged. There are many paths, through which fluids may pass, in the porous film 111a, and when one of the paths is enlarged, a surface of the fluid path with negative charges (bound anions) may be in a pseudostate in which the negative charges are balanced by mobile cations having positive charges. In this state, when a positive voltage is applied to the first electrode 111b and a negative voltage is applied to the second electrode 111c, negative pressure is generated, and the fluid inside the driver 111 moves in the direction ① due to the negative pressure. In this case, a drug is sucked through an inflow path 141 and flows into the chamber 112 through an inflow valve 140. In this case, an outflow valve 150 is closed such that the negative pressure is not transferred to an outflow path 151. In contrast to this, when a negative voltage is applied to the first electrode 111b and a positive voltage is applied to the second electrode 111c, the positive pressure in an opposite direction is generated by a reversible electrochemical reaction. The fluid inside the driver 111 moves in the direction ② by the positive pressure. In this case, the drug stored in the chamber 112 is introduced into a target through the discharge valve 150 and the discharge path 151. In this case, the inflow valve 140 is blocked such that the positive pressure is not transferred to the inflow path 141.

This phenomenon is called an electroosmotic phenomenon, and a pump that uses the principle is the electroosmotic pump 110.

An electrode used for the driver 111 may be implemented by a platinum mesh such as a porous electrode, porous carbon paper or fiber, or various electrode materials applied onto a porous structure so as to smoothly move a fluid.

In addition, the electrode used for the driver 111 may be coated with various fixable materials, such as an impermeable substrate material, by drop coating or spin coating. In this case, the impermeable substrate material is a plate-shaped substrate including at least one of a conductive material, a semiconductor material, and a non-conductive material, and an electrode material applied thereon may include metal, metal oxide, conducting polymer, metal hexacyanoferrate, a carbon nanostructure, or a composite thereof.

The driver 111 alternately supplies polarities of voltages or polarities of currents to the first electrode 111b and the second electrode 111c, and thereby, an electrochemical reaction occurs reversibly in both forward and reverse directions. As the electrochemical reaction occurs repeatedly in the forward and reverse directions, a fluid inside the electroosmotic pump repeatedly performs a reciprocating motion. In addition, the fluid in the first electrode 111b and the second electrode 111c is consumed and regenerated repeatedly by the reversible electrochemical reaction in the repeated forward and reverse directions, When the inflow valve 140 and the discharge valve 150 are coupled to the chamber 112 of the electroosmotic pump 110, the drug in the drug storage 120 is sucked through the inflow path 141 during an inflow operation and stored in the chamber 112 through the inflow valve 140. In addition, during an outflow operation, the drug stored in the chamber 112 is discharged to the insertion unit 130 through the discharge valve 150 and the discharge path 151.

Therefore, the pressure generated by the electroosmotic pump 110 and the volume of a discharged drug may be adjusted by controlling the magnitude and application time of a voltage or a current applied to the first and second electrodes 111b and 111c.

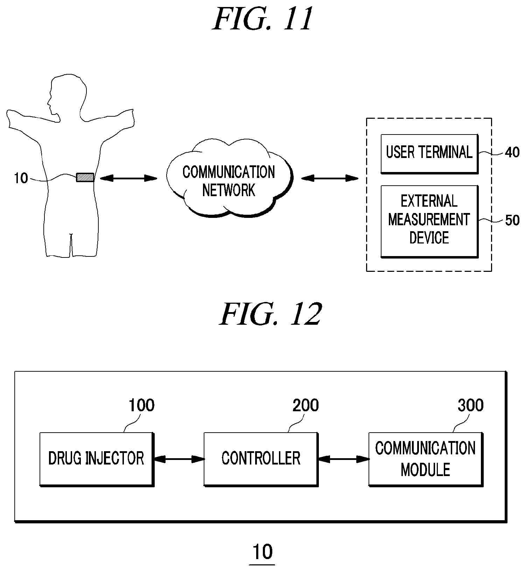

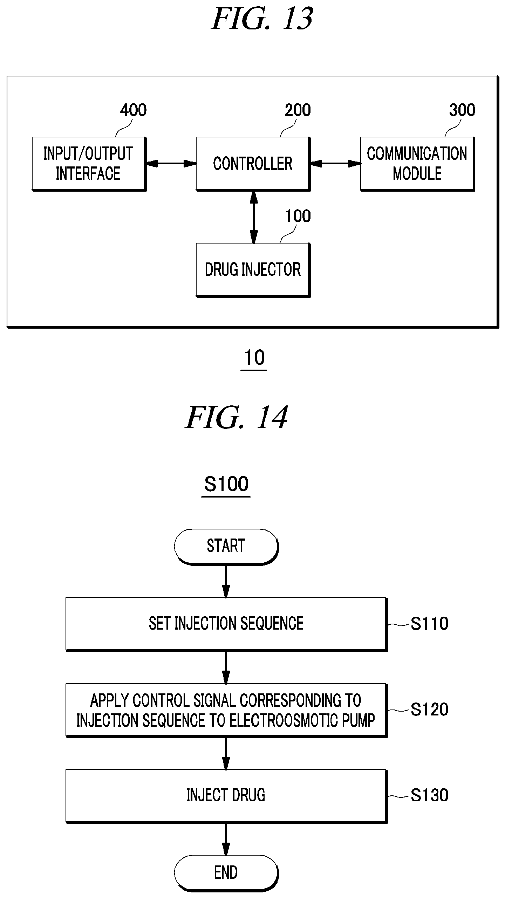

FIG. 11 is an application example diagram of a drug injection device according to an embodiment of the present invention, FIG. 12 is a block diagram schematically illustrating a configuration of the drug injection device illustrated in FIG. 11, and FIG. 13 is a block diagram schematically illustrating the configuration of the drug injection device illustrated in FIG. 11. An operation of the drug injection device 10 is specifically described with reference to FIG. 11 to FIG. 13.

The drug injection device 10 receives predetermined information from a user and sets a speed-based injection sequence based on the information, and a process of setting the speed-based injection sequence may be performed according to data used to set the speed-based injection sequence. Hereinafter, the process of setting the speed-based injection sequence is described.

The process of setting, by the drug injection device 10, the speed-based injection sequence according to an embodiment of the present invention is described with reference to FIG. 12.

The drug injection device 10 may set the speed-based injection sequence by using a drug injection condition. Here, the drug injection condition includes a drug injection period and a drug injection speed. A user may distinguish multiple segments, each having a predetermined duration for the drug injection period, and input the drug injection condition by setting a drug injection speed for each segment. Then, the drug injection device 10 sets a speed-based injection sequence for the conditions set for each segment.

The drug injection device 10 may further include a communication module 300 to receive a drug injection condition from the user terminal 40. The drug injection condition is received from a user through the user terminal 40 that is communicably connected to the drug injection device 10 through the communication module 300. Then, the controller 200 sets a speed-based injection sequence corresponding to the drug injection condition and transmits a control signal to the drug injector 100. Here, the speed-based injection sequence is not set by using the drug injection condition but may be directly selected by a user through the user terminal 40 or may be received by being calculated by an external computing device based on the user's past use history of the drug injection device.

The user terminal 40 may include a computer or portable terminal that may be connected to the drug injection device 10 through wireless communication. Here, the computer includes, for example, a desktop computer, a laptop computer, or so on in which a web browser is stored, and the portable terminal is, for example, a wireless communication device that ensures portability and mobility and may include all kinds of handheld-based wireless communication devices, such as various smartphones, tablet personal computers (PCs), and smart watches. The user terminal 40 is managed by a wearer of the drug injection device 10 or a medical professional, and may set a drug injection condition through an application running on the user terminal 40.

In addition, the drug injection device 10 may set the speed-based injection sequence by using user input data. Here, the user input data includes a user's blood sugar level or the user's meal information. The user input data is received from a user through the user terminal 40 connected to the drug injection device 10 through the communication module 300, and the controller 200 calculates the drug injection condition based on the received user input data. In addition, a speed-based injection sequence corresponding to the drug injection condition is set, and a control signal corresponding to the speed-based injection sequence is transmitted to the drug injector 100.

In addition, the drug injection device 10 may set the speed-based injection sequence by using user's biometric measurement data. Here, the user's biometric measurement data includes a user's blood sugar information. The user's biometric measurement data is received through an external measurement device 50 connected to the drug injection device 10 through the communication module 300, and the controller 200 calculates a drug injection condition based on the received user's biometric measurement data. In addition, a speed-based injection sequence corresponding to the drug injection condition is set, and a control signal corresponding to the speed-based injection sequence is transmitted to the drug injector 100. Here, the external measurement device 50 may be a device that senses a user's blood sugar level.

Next, a process of setting a speed-based injection sequence by the drug injection device is described with reference to FIG. 13.

The drug injection device 10 may set the speed-based injection sequence by using a drug injection condition. The drug injection device 10 may further include a user input/output interface module 400, and receives a drug injection condition from a user through the user input/output interface module 400. In an embodiment in which the drug injection device 10 includes the user input/output interface module 400, a user may control an operation of the drug injection device 10 by directly inputting a drug injection condition and so on to the user input/output interface module 400 without separately using the user terminal 40. In this case, the communication module 300 may be optionally excluded.

The controller 200 sets a speed-based injection sequence corresponding to the drug injection conditions input through the user input/output interface module 400 and transmits a control signal corresponding to the speed-based injection sequence to the drug injector 100. Here, the speed-based injection sequence may be directly selected by a user through the user input/output interface module 400 without being set by using the drug injection condition.

The user input/output interface module 400 may include a physical input/output button coupled to an external housing including the drug injection device 10, and a signal processing circuit that transmits a signal generated according to an operation of the physical input/output button to the controller 200.

Alternatively, the user input/output interface module 400 may output a user interface (UI) that guides a user to input information on a drug injection condition or speed-based injection sequence through a display including a touch screen.

Additionally, in the process of setting the speed-based injection sequence described above, the controller 200 may also set a speed-based injection sequence corresponding to each drug injection condition by referring to a table in which multiple speed-based injection sequences are stored for each drug injection condition.

FIG. 14 is a flowchart illustrating a drug injection method according to an embodiment of the present invention.

Referring to FIG. 1, FIG. 2, and FIG. 14, a drug injection method S100 according to an embodiment of the present invention includes step S110 of setting a speed-based injection sequence for causing the drug injection device 10 to operate in a basal injection mode, and step S120 of applying a control signal corresponding to the speed-based injection sequence to the electroosmotic pump 110. Thereafter, in response to the control signal, the electroosmotic pump 110 alternately generates negative pressure and positive pressure for each pulse block to suck and discharge a drug and inject the drug into a user (step S130). Here, the speed-based injection sequence includes at least one pulse block defining a voltage pulse or current pulse to be applied to the electroosmotic pump 110 and at least one pause block maintaining a voltage of 0 volt or a current of 0 ampere for a predetermined time after the pulse block is applied, and each pulse block defines a pair of pulse signals including a forward pulse and a reverse pulse that are applied to the electroosmotic pump 110 to alternately generate the negative pressure and positive pressures.

Hereinafter, each step is described in detail.

In the process (step S110) of setting the speed-based injection sequence, the drug injection device (10) may set the speed-based injection sequence by using a drug injection condition, and may have various embodiments depending on when the drug injection condition is input. Here, the drug injection condition includes a drug injection period and a drug injection speed, or a drug injection amount and a drug injection period.

The drug injection device 10 may receive the drug injection condition from a user through the user terminal 40 connected to the drug injection device 10 through the communication module 300. Then, the controller 200 sets a speed-based injection sequence corresponding to the drug injection condition and transmits a control signal corresponding the speed-based injection sequence to the drug injector 100. Here, the speed-based injection sequence may be directly selected by a user through the user terminal 40 without being set by using the drug injection condition.

In addition, the drug injection device 10 may receive the drug injection condition from the user through the user input/output interface module 400, and the controller 200 sets a speed-based injection sequence corresponding to the drug injection condition and transmits a control signal to the drug injector 100. Here, the speed-based injection sequence may be directly selected by a user through the user input/output interface module 400 without being set by using the drug injection condition.

In addition, the drug injection device 10 may set the speed-based injection sequence by using user input data. Here, the user input data includes a user's blood sugar level or a user's meal information. The user input data is received from a user through the user terminal 40 communicatively connected to the drug injection device 10 through the communication module 300, and the controller 200 calculates the drug injection condition based on the received user input data. In addition, the speed-based injection sequence corresponding to the drug injection condition is set, and the control signal corresponding to the speed-based injection sequence is transmitted to the drug injector 100.

In addition, the drug injection device 10 may set the speed-based injection sequence by using a user's biometric measurement data. Here, the user's biometric measurement data includes a user's blood sugar level information. The user's biometric measurement data is received through the external measurement device 50 communicatively connected to the drug injection device 10 through the communication module 300, and the controller 200 calculates the drug injection condition based on the received user's biometric measurement data. In addition, the speed-based injection sequence corresponding to the drug injection condition is set, and the control signal corresponding to the speed-based injection sequence is transmitted to the drug injector 100. Here, the external measurement device 50 may be a device that senses a user's blood sugar level.

Meanwhile, in the process of setting the speed-based injection sequence, the controller 200 may set the speed-based injection sequence corresponding to the drug injection condition by referring to a table in which multiple speed-based injection sequences are stored for each drug injection condition.

In addition, in a process of applying a control signal to the electroosmotic pump (step S120), the control signal corresponding to a speed-based injection sequence may include a voltage pulse pair including a forward voltage pulse and a reverse voltage pulse or a current pulse pair including a forward current pulse and a reverse current pulse in pulse block units, and in response to the control signal, the electroosmotic pump 110 may perform an operation of alternately generating negative pressure and positive pressure for each pulse block to suck and discharge a drug (step S130), thereby injecting the drug into a user.

A method according to an embodiment of the present disclosure may be performed in the form of a recording medium including instructions executable by a computer, such as a program module executed by a computer. A computer readable medium may be any available medium that may be accessed by a computer and includes both volatile and nonvolatile media, removable and non-removable media. Also, the computer readable medium may include a computer storage medium. A computer storage medium includes both volatile and nonvolatile media and removable and non-removable media implemented by any method or technology for storing information, such as computer readable instructions, data structures, program modules or other data.

In addition, although the method and system of the present disclosure are described with respect to specific embodiments, some or all of components or operations thereof may be implemented by using a computer system having a general-purpose hardware architecture.

those skilled in the art to which the present disclosure belongs will understand that the present disclosure may be easily modified into another specific form based on the descriptions given above without changing the technical idea or essential features of the present disclosure. Therefore, the embodiments described above should be understood as illustrative in all respects and not limiting. The scope of the present invention is indicated by the claims described below, and all changes or modified forms derived from the meaning, scope of the claims, and their equivalent concepts should be interpreted as being included in the scope of the present invention.

The scope of the present application is indicated by the claims described below rather than the detailed description above, and all changes or modified forms derived from the meaning, scope of the claims, and their equivalent concepts should be interpreted as being included in the scope of the present application.

## Claims

1. A drug injection device comprising:
a drug injector configured to suck a drug from a drug storage by electrochemically operating an electroosmotic pump and discharge an sucked drug to an injection target; and
a controller configured to output, to the drug injector, a control signal corresponding to a speed-based injection sequence for causing the electroosmotic pump to operate in a basal infusion mode,
wherein the speed-based injection sequence includes at least one pulse block defining one of a voltage pulse and a current pulse applied to the electroosmotic pump and at least one pause block in which one of a voltage of 0 volt and a current of 0 ampere is maintained for a predetermined period of time after the pulse block is applied,
the at least one pulse block defines a pair of pulse signals including a forward pulse and a reverse pulse that are applied to the electroosmotic pump to alternately generate negative pressure and positive pressure, and
the electroosmotic pump alternately generates negative pressure and positive pressure for each pulse block to suck and discharge the drug.

2. The drug injection device of claim 1, wherein
in the speed-based injection sequence, multiple pulse blocks satisfying a drug injection speed are arranged according to a drug injection condition including a drug injection period and the drug injection speed, and
a sum of durations of the multiple pulse blocks and durations of multiple pause blocks respectively arranged after the multiple pulse blocks is set to correspond to the drug injection period.

3. The drug injection device of claim 1, wherein
in the speed-based injection sequence, the at least one pulse block and the at least one pause block are arranged for a unit time, and
the controller repeatedly provides the speed-based injection sequence during a drug injection period.

4. The drug injection device of claim 2, wherein
in the speed-based injection sequence, the durations of the multiple pause blocks are set to be less than a recommended pause period.

5. The drug injection device of claim 2, wherein
the speed-based injection sequence is arranged such that a pulse block supplying a maximum drug amount among the multiple pulse blocks is output first.

6. The drug injection device of claim 1, wherein
M (M is a natural number less than or equal to N that is a natural number) pulse blocks selected from among N pulse blocks that supply different amounts of drugs are arranged in the speed-based injection sequence according to a drug injection condition,
the drug injection condition includes a drug injection period and a drug injection speed, and
a sum of durations of the selected M pulse blocks and durations of multiple pause blocks respectively arranged after the M pulse blocks is set to correspond to the drug injection period.

7. The drug injection device of claim 1, wherein
the speed-based injection sequence is set such that a sum of number of uses of selected pulse blocks is a preset number or a maximum number, while the M pulse blocks satisfy a drug injection speed of a drug injection condition, and a duration of the at least one block arranged after the at least one pulse block is less than a recommended pause period.

8. The drug injection device of claim 2, wherein
in the speed-based injection sequence, the multiple pulse blocks and the multiple pause blocks respectively assigned multiple segments are arranged such that drug injection speeds of some segments are equal to each other and drug injection speeds of the other segments are different from each other depending on segments in which the drug injection periods are divided for each preset time units.

9. The drug injection device of claim 1, wherein
the at least one pulse block and the at least one pause block form one group of which duration is set to a first time, and
a duration of the at least one pause block is set to a second time obtained by subtracting a duration of the at least one pulse block from the first time.

10. The drug injection device of claim 1, wherein
the pair of pulse signals includes a pair of voltage pulses including a forward voltage pulse and a reverse voltage pulse and includes a stabilization pulse that maintains a voltage of o V for a predetermined time after application of the forward voltage pulse and the reverse voltage pulse, or
includes a current pulse pair including a forward current pulse and a reverse current pulse and includes a stabilization pulse that maintains a current of 0 A for a predetermined time after application of the forward current pulse and the reverse current pulse.

11. The drug injection device of claim 1, wherein the electroosmotic pump includes:
a driver configured to alternately generate positive pressure and negative pressure by being electrochemically driven by the control signal; and
a chamber configured to suck the drug from a drug source according to pressure of a driver and discharge the drug to an insertion unit.

12. The drug injection device of claim 1, wherein
the drug injection device receives a drug injection condition from one of a user terminal and a user input/output interface module, and
the controller outputs the control signal by using a speed-based injection sequence corresponding to the drug injection condition including a drug injection period and the drug injection speed.

13. The drug injection device of claim 1, wherein
the drug injection device receives user input data from a user terminal,
the controller calculates a drug injection condition based on user input data and outputs the control signal by using a speed-based injection sequence corresponding to the drug injection condition,
the user input data includes a blood sugar level, activity information, or meal information of a user, and
the drug injection condition includes a drug injection period and a drug injection speed.

14. The drug injection device of claim 1, wherein
the drug injection device receives biometric data of a user from an external measurement device,
the controller calculates a drug injection condition based on the biometric data of the user and outputs the control signal by using a speed-based injection sequence corresponding to the drug injection condition,
the biometric data of the user includes blood sugar information or activity information of the user, and
the drug injection condition includes a drug injection period and a drug injection speed.

15. The drug injection device according to any one of claims 12 to 14, wherein
the controller sets the speed-based injection sequence corresponding to the drug injection condition by referring to a table in which multiple speed-based injection sequences are stored for each drug injection condition.

16. The drug injection device of claim 1, wherein
the drug injection device receives a speed-based injection sequence set by one of a user terminal and a user input/output interface module, and
the controller outputs the control signal by using the speed-based injection sequence.

17. The drug injection device of claim 1, wherein
the speed-based injection sequence is calculated and received through an external computing device based on a user's past use history of the drug injection device.

18. A drug injection method of a drug injection device including an electroosmotic pump, the drug injection method comprising:
setting a speed-based injection sequence for causing the drug injection device to operate in a basal infusion mode;
applying a control signal corresponding to the speed-based injection sequence to the electroosmotic pump; and
causing the electroosmotic pump to alternately generate negative pressure and positive pressure for each pulse block in response to the control signal such that the drug is sucked and discharged,
wherein the speed-based injection sequence includes at least one pulse block defining one of a voltage pulse and a current pulse applied to the electroosmotic pump and at least one pause block in which one of a voltage of 0 volt and a current of 0 ampere is maintained for a predetermined period of time after the pulse block is applied, and
the at least one pulse block defines a pair of pulse signals including a forward pulse and a reverse pulse that are applied to the electroosmotic pump to alternately generate the negative pressure and the positive pressure.

19. The drug injection method of claim 18, wherein
in the setting of the speed-based injection sequence, multiple pulse blocks satisfying a drug injection speed are arranged according to a drug injection condition including a drug injection period and the drug injection speed, and a sum of durations of the multiple pulse blocks and durations of multiple pause blocks respectively arranged after the multiple pulse blocks is set to correspond to the drug injection period.

20. The drug injection method of claim 18, wherein
in the setting of the speed-based injection sequence, the at least one pulse block and the at least one pause block are arranged for a unit time to set the speed-based injection sequence.

21. The drug injection method of claim 19, wherein
in the setting of the speed-based injection sequence, the durations of the multiple pause blocks are set to be less than a recommended pause period.

22. The drug injection method of claim 19, wherein
in the setting of the speed-based injection sequence, the speed-based injection sequence is set such that a pulse block supplying a maximum drug amount among the multiple pulse blocks is output first.

23. The drug injection method of claim 18, wherein
in the setting of the speed-based injection sequence, M (M is a natural number less than or equal to N that is a natural number) pulse blocks selected from among N pulse blocks that supply different amounts of drugs are arranged according to a drug injection condition to set the speed-based injection sequence,
the drug injection condition includes a drug injection period and a drug injection speed, and
a sum of durations of the selected M pulse blocks and durations of multiple pause blocks respectively arranged after the M pulse blocks is set to correspond to the drug injection period.

24. The drug injection method of claim 23, wherein
in the setting of the speed-based injection sequence, the speed-based injection sequence is set such that a sum of number of uses of selected pulse blocks is a preset number or a maximum number while the M pulse blocks satisfy a drug injection speed of a drug injection condition and a duration of the at least one block arranged after the at least one pulse block is less than a recommended pause period.

25. The drug injection method of claim 19, wherein
in the setting of the speed-based injection sequence, the multiple pulse blocks and the multiple pause blocks respectively assigned multiple segments are arranged such that drug injection speeds of some segments are equal to each other and drug injection speeds of the other segments are different from each other depending on segments in which the drug injection periods are divided for each preset time units.

26. The drug injection method of claim 18, wherein
in the setting of the speed-based injection sequence, the speed-based injection sequence is set such that an amount of sucked drugs is equal to an amount of discharged drugs in response to a pair of voltage pulse signals or a pair of current pulse signals by controlling magnitudes and durations of the pair of voltage pulse signals or magnitudes and durations of the pair of current signals.

27. The drug injection method of claim 18, wherein
the pair of pulse signals includes a pair of voltage pulses including a forward voltage pulse and a reverse voltage pulse and includes a stabilization pulse that maintains a voltage of o V for a predetermined time after application of the forward voltage pulse and the reverse voltage pulse, or includes a current pulse pair including a forward current pulse and a reverse current pulse and includes a stabilization pulse that maintains a current of 0 A for a predetermined time after application of the forward current pulse and the reverse current pulse.

28. The drug injection method of claim 18, wherein
in the setting of the speed-based injection sequence, the drug injection device sets the speed-based injection sequence corresponding to a drug injection condition received from one of a user terminal and a user input/output interface module, and
the drug injection condition includes a drug injection period and a drug injection speed.

29. The drug injection method of claim 18, wherein
in the setting of the speed-based injection sequence, the drug injection device calculates a drug injection condition based on user input data received from a user terminal, and sets the speed-based injection sequence corresponding to the drug injection condition,
the user input data includes a blood sugar level of a user and user activity information of the user, and
the drug injection condition includes a drug injection period and a drug injection speed.

30. The drug injection method of claim 18, wherein
in the setting of the speed-based injection sequence, the drug injection device calculates a drug injection condition based on user's biometric measurement data received from an external measurement device, and sets a speed-based injection sequence corresponding to the drug injection condition,
the user's biometric measurement data includes blood sugar information of a user or activity information of the user, and
the drug injection condition includes a drug injection period and a drug injection speed.

31. The drug injection method according to any one of claims 28 to 30, wherein
in the setting of the speed-based injection sequence, the speed-based injection sequence corresponding to the drug injection condition by referring to a table in which multiple speed-based injection sequences are stored for each drug injection condition.

32. The drug injection method of claim 18, wherein
in the setting of the speed-based injection sequence, the speed-based injection sequence is set by receiving a speed-based injection sequence set by one of a user terminal and a user input/output interface module.

33. The drug injection method of claim 18, wherein
in the setting of the speed-based injection sequence, the speed-based injection sequence is set by receiving, from an external computing device, a speed-based injection sequence calculated from a user's past use history of the drug injection device.
